# EUROPEAN PATENT APPLICATION

(11) **EP 2 460 546 A1**
(43) Date of publication of application: **06.06.2012**
(21) Application number: 10193242.4
(22) Date of filing: 01.12.2010
(51) Int. Cl.: A61M 5/142

(54) **Ambulatory infusion device and method for acoustically signaling numerical values**

(71) Applicant: F. Hoffmann-La Roche AG, 4070 Basel (CH); Roche Diagnostics GmbH, 68305 Mannheim (DE)
(72) Inventor: Bernini, Nicole, 3400 Burgdorf (CH); Sigrist, Reto, 3207 Golaten (CH); Dueringer, Daniel, 4656 Starrkirch-Wil (CH); Schrotberger, Reto, 3012 Bern (CH); Luder, Pascal, 3400 Burgdorf (CH); Kompis, Martin, 3052 Zollikofen (CH)
(74) Representative: Rentsch Partner AG

(57) **Abstract**

An ambulatory infusion device (1) comprises an audio encoder (12) connected to an electro-acoustic transducer (11). The audio encoder (12) is configured to generate an acoustically reproducible encoding of numerical values by representing each digit with a sequence of successive notes of a musical scale (2). The number of notes in the sequence corresponds to the number value of the respective digit. Subsequently, the numerical values are signaled via the electro-acoustic transducer (11) using the generated encoding. Representing a numerical digit with a sequence of successive notes of a musical scale, where the number of notes in the sequence corresponds to the number value of the digit, has the advantage that numerical values can be acoustically signaled to a user in a very intuitive fashion, enabling users with a visual impairment to easily and intuitively program and operate the ambulatory infusion device (1).

## Description

### Field of the Invention

The present invention relates to an ambulatory infusion device and a method for signaling acoustically numerical values to a user of the ambulatory infusion device. Specifically, the present invention relates to an ambulatory infusion device comprising an audio encoder for generating an acoustically reproducible encoding of numerical values, and for signaling the numerical values via an electro-acoustic transducer.

### Background of the Invention

External ambulatory infusion devices for the infusion of a liquid drug over an extended time period are known in the art for a number of therapies. In particular, such devices form the basis for a state-ofthe-art therapy of Diabetes Mellitus by CSII (Continuous Subcutaneous Insulin Infusion). An example of such an ambulatory infusion device is disclosed in WO 2003053498 A2 to which reference is made for a typical design and typical features of such devices. Besides diabetes therapy, these devices may be used for a number of further therapies, such as cancer treatment or pain therapy, without requiring substantial modification.

Continuous subcutaneous insulin infusion using an ambulatory infusion device, i.e. an infusion pump, more closely mimics a healthy (normal) pancreas than multiple insulin injections. It is thus an effective, and often a preferred, means of maintaining normal blood glucose levels. However, ambulatory infusion devices for insulin infusion are typically not fully accessible to blind or visually impaired people or to people who have hearing loss. These, handicaps, in particular visual impairment or even blindness, are typical long-term damages resulting from diabetes. Although, today's insulin pumps do provide some limited audio output, it is generally found to be of minimal use to people with the above mentioned handicap(s).

WO 00/10628 describes an RF programming device for an external infusion device. The programming device includes a receiver and provides an audio indication that commands have been received and acknowledged by the external infusion device. For programming an insulin bolus up and down arrow keys are pressed to increment or decrement the amount of insulin in defined units. Counting the bolus increments is facilitated by accompanying presses of the up and down arrow keys with beeps of varying audio tones. A defined number of notes belonging to a musical chord will be used in repeating sequence as the up and down arrow keys are repeatedly pressed to select a desired bolus amount. For example, the first key press of the up arrow key, for setting a bolus increment in the external infusion device, will be accompanied by the first note in the chord, while the second press will be accompanied by the second note in the chord. For a chord with four notes, for example, the audio sequence will be repeated for the fifth press of the up arrow key, starting again with the first note of the chord. When the desired bolus amount is programmed, the user presses an activate key and the external infusion device confirms the bolus amount with a series of audible beeps. Specifically, the external infusion device plays back the beep sequence generated during the bolus amount selection. Playing back the beep sequence generated during the bolus amount selection may provide to the user some useful audible feedback when small amounts with few incremental steps were entered. However, for large amounts with many incremental steps and/or when a combination of up and down keys was used, e.g. for correction purposes, the beep sequence may become too long and/or complicated for some users to reliably determine the specified amount.

US 7,515,060 describes an insulin pump for the visually impaired which generates audible sounds to assist the user in navigating through pump menus. The sounds indicate the current screen displayed by the pump and also signify buttons pressed on the pump. Beeps with different pitch and/or number of repetition are used to identify menu screens programmed into the pump. Sounds with different pitch are also associated with the up and down keys on the pump to assist in setting pump delivery rates, times, and other settings. According to US 7,515,060, sounds of increasing pitch are associated with numerical values scrolled through using the up and down keys, such that lower values are associated with sounds of a lower pitch, and higher values are associated with sounds of a higher pitch. Additional unique sounds are used to denote a position within the range scrolled through, for example, the pump emits a unique sound each time a value is reached that is a multiple of ten. Sounds may further be associated with alerts and/or alarms, and the pump emits sounds, such as numerical values or messages, in an audibly encoded format, e.g. in Morse code. As audible Morse code is based on a series of tones having the same pitch but different lengths, it may be very useful for transmissions over communication channels with heavy disturbance and/or interference; however, some users may not find it intuitive and have troubles to reliably determine the specified amount.

### Summary of the Invention

It is an object of this invention to provide an ambulatory infusion device and a method for signaling acoustically numerical values to a user of the ambulatory infusion device, which device and method do not have at least some of the disadvantages of the prior art. In particular, it is an object of the present invention to provide an ambulatory infusion device and a method for signaling acoustically numerical values without the need of playing back lengthy beep sequences generated during entry of the numerical values, and without having to rely on Morse code.

According to the present invention, these objects are achieved through the features of the independent claims. In addition, further advantageous embodiments follow from the dependent claims and the description.

According to the present invention, the above-mentioned objects are particularly achieved in that an ambulatory infusion device, which comprises an electro-acoustic transducer, is further provided with and an audio encoder connected to the electro-acoustic transducer and configured receive numerical values and to generate subsequently an acoustically reproducible encoding of numerical values by representing a digit of a numerical value with a sequence of successive notes of a musical scale, the sequence comprising a quantity of notes corresponding to a value of the digit, and to signal the numerical values via the electro-acoustic transducer. Preferably, the numerical values are received and stored in the ambulatory infusion device, and, subsequently, the audio encoder retrieves the numerical values stored in the ambulatory infusion device and generates the acoustically reproducible encoding from the numerical values previously entered and stored in the ambulatory infusion device. Thus, the acoustic rendering of the numerical values is performed not as a feedback during data entry, but after entry of the numerical value is complete. Rendering numerical values acoustically by representing each digit of the numerical value with a sequence of successive notes of a musical scale, where the number of notes in the sequence corresponds to the number value of the digit, has the advantage that single or multi-digit numerical values can be signaled acoustically to a user in a very intuitive fashion with only a small technical overhead. Thus, the present disclosure allows consistent signalization of any kind of numerical information or data. This is especially favorable over prior art devices that rely on acoustical encoding of key presses or numerical increments. Consequently, it is possible to provide the ambulatory infusion device with an improved user interface which enables a user who has visual impairment to easily and intuitively program, operate, and obtain feedback from the ambulatory infusion device.

In an embodiment, the ambulatory infusion device further comprises different user selectable acoustic profiles, each acoustic profile defining the pitch level of the scale, and the audio encoder is configured to signal the numerical values using the pitch level defined in the acoustic profile selected by the user. Thus, for users with a partial hearing loss handicap, the numerical values can be rendered acoustically with lower frequencies than for users without such a hearing loss handicap, thereby enabling also users with a partial hearing loss to easily and intuitively program, operate, and obtain feedback from the ambulatory infusion device.

In another embodiment, the different acoustic profiles further define different durations of the notes and the audio encoder is configured to signal the numerical values using the durations of the notes defined in the acoustic profile selected by the user.

Preferably, the audio encoder is further configured to generate an acoustically reproducible encoding of numerical values having more than one digit, by generating a pause of defined duration between representations of two successive digits.

In yet another embodiment, the audio encoder is further configured to generate an acoustically reproducible encoding of a numerical infusion amount value having an integer part and a fractional part, by generating a defined tone between representations of the integer part and the fractional part.

In another embodiment, the audio encoder is further configured to generate an acoustically reproducible encoding of numerical time of day values having a two-digit hours part and a two-digits minutes part, by generating a defined tone between representations of the hours part and the minutes part.

In addition to an ambulatory infusion device and a method for signaling acoustically numerical values to a user of the ambulatory infusion device, the present invention also relates to a computer program product comprising a tangible computer readable medium having stored thereon computer program code configured to direct a processor of the ambulatory infusion device or of a remote controller for a dosing unit, which in combination form the ambulatory infusion device. Specifically, the computer program code is configured to direct the processor such that ambulatory infusion device (or the remote controller of the dosing unit) generates an acoustically reproducible encoding of a numerical value previously entered and/or stored in the ambulatory infusion device (or the remote controller of the dosing unit), by representing a digit of the numerical value with a sequence of successive notes of a musical scale, the sequence comprising a quantity of notes corresponding to a value of the digit, and signals the numerical value via an electro-acoustic transducer.

### Brief Description of the Drawings

The present invention will be explained in more detail, by way of example, with reference to the drawings in which:
- Figure 1:: shows a block diagram illustrating schematically an ambulatory infusion device with an electro-acoustic transducer and an audio encoder for generating an acoustically reproducible encoding of numerical values.
- Figure 2:: shows an example of a musical scale, specifically, a one-octave C major scale.
- Figure 3:: shows a flow diagram illustrating an exemplary sequence of steps for signaling acoustically numerical values to a user of the ambulatory infusion device.
- Figure 4:: shows a flow diagram illustrating another exemplary sequence of steps for signaling acoustically numerical values to a user of the ambulatory infusion device.

### Detailed Description of the Preferred Embodiments

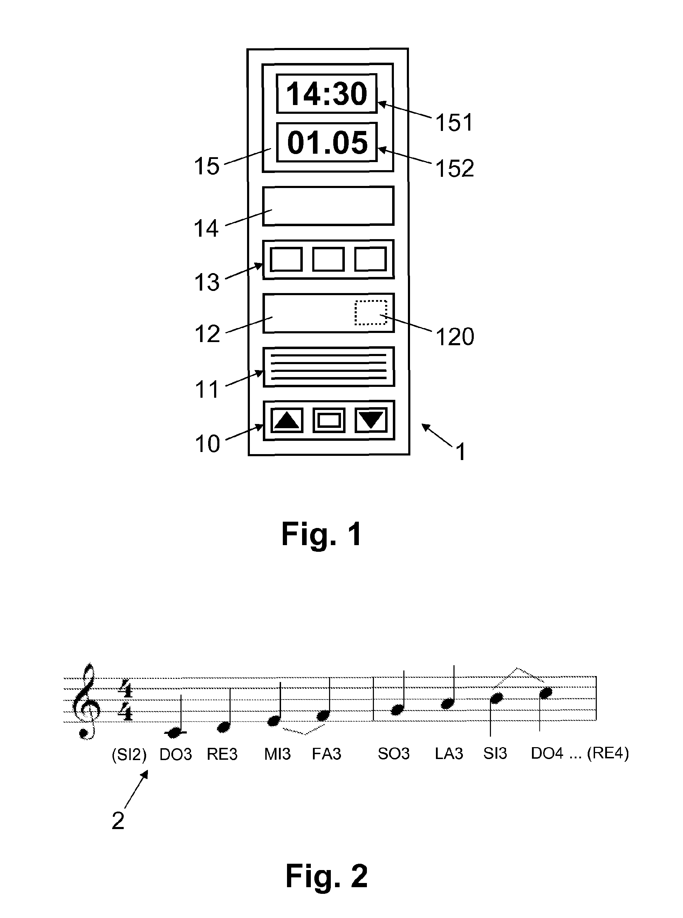

In Figure 1, reference numeral 1 refers to an ambulatory infusion device, particularly an ambulatory infusion device for the infusion of a liquid drug, e.g. insulin, over an extended time period for therapeutic purposes, e.g. for therapy of Diabetes Mellitus by Continuous Subcutaneous Insulin Infusion (CSII). Alternatively, reference numeral 1 refers to a remote controller for a dosing unit, wherein the remote controller and the dosing unit, in combination, form an ambulatory infusion device.

As illustrated schematically in Figure 1, the ambulatory infusion device 1 comprises several hardware components including operating elements 10, e.g. data entry keys, up/down-keys, a scroll wheel, or the likes, an electro-acoustic transducer 11, e.g. a loudspeaker, a vibrator, an infusion pump, a programmable processor with data and program memory, a display 15, an illumination device, and an energy storage, e.g. one or more disposable and/or rechargeable batteries, etc., typically in one common housing.

The ambulatory infusion device 1 further comprises functional modules, including an audio encoder 12 and a control module 14, which are implemented preferably by way of programmed software modules. The software modules include program code for controlling the processor. The program code is stored on a tangible computer-readable medium connected fixed or removably to the processor. One skilled in the art will understand, however, that in alternative embodiments, the functional modules can be implemented fully or at least partly by way of hardware components.

The control module 14 is configured to process user data entries, visualize and show information on the display 15, control insulin infusion, monitor energy supply, generate acoustic, vibration, and visual alarms, etc.

The control module 14 is further configured to receive from a user a selection of one of several acoustic profiles 13 stored in the ambulatory infusion device 1. The acoustic profiles 13 define different pitch levels for musical scales and/or different durations of the notes of the musical scale. Furthermore, the acoustic profiles 13 define pitch levels and/or durations of various acoustic signals for confirming and indicating acoustically to a user activated operating elements, current cursor and/or menu positions, selected or highlighted menu functions, parameters 151, 152, etc. It should be stated that a musical scale is typically an ordered sequence of successive notes with consistently increasing or decreasing pitch, different musical scales are defined in different cultures. To improve intuitiveness, a musical scale is selected which is familiar to the user. One skilled in the art will understand however, that instead of a conventional, defined, i.e. "standard", musical scale, such as, for example, the C-major scale, any other scale with an ordered sequence of successive tones with consistently increasing or decreasing pitch could be selected, used and/or specified in the acoustic profiles 13.

As will be explained below in more detail, the audio encoder 12 is connected to the electro-acoustic transducer 11 and configured to encode numerical values of parameters entered and/or stored in the ambulatory infusion device 1 for rendering via the electro-acoustic transducer 11. Specifically, the audio encoder 12 is configured to generate an acoustically reproducible encoding of the numerical values, and to signal the numerical values via the electro-acoustic transducer 11, i.e. to reproduce or render the numerical values acoustically, based on the encoding. For example, the numerical values include time values, indicating time durations or points in time, i.e. the time of day. For example, time durations include one or more digits representing a number of hours and/or minutes; and the time of day includes a two-digit hours part "HH" and a two-digits minutes part "MM", e.g. separated by a colon, e.g. "14:30", as illustrated in Figure 1 for parameter 151 shown on display 15. The numerical values further include the values of other parameters representative of insulin amounts associated with a one shot or extended bolus. Preferably, the numerical values of insulin amounts include a (one or two-digit) integer part "NN" and a fractional part "DD", e.g. separated by a comma or period, e.g. "01:05", as illustrated in Figure 1 for parameter 152 shown on display 15. In the present description, numerical values have one or more decimal digits; however, one skilled in the art will understand that different numerical systems could be used, without deviating from the scope of the invention.

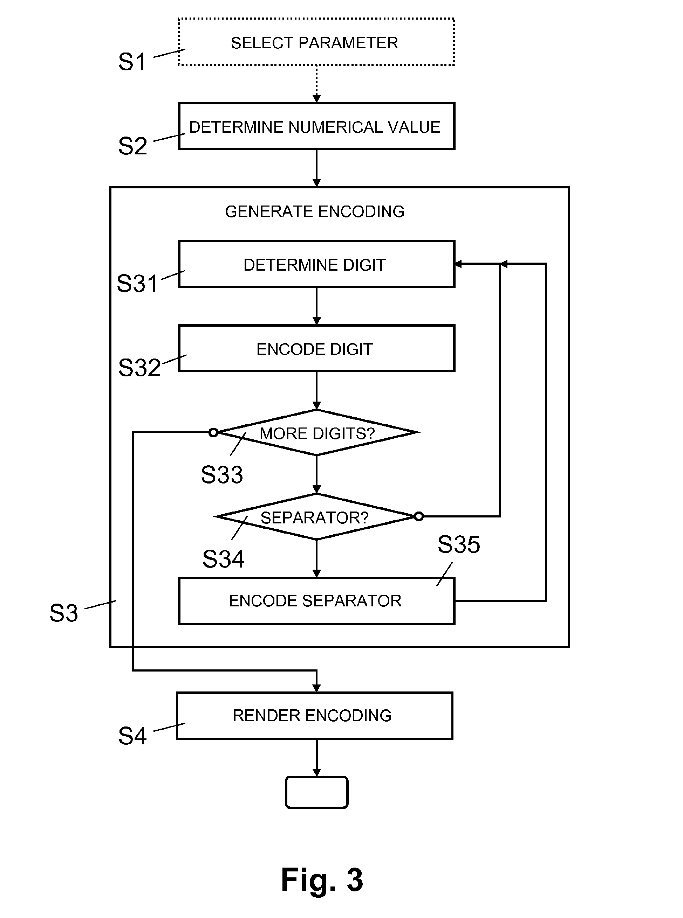

In the following paragraphs, described with reference to Figures 2 and 3 are possible sequences of steps performed by the control module 14 or audio encoder 12, respectively, for signaling acoustically numerical values to the user of the ambulatory infusion device 1, by the audio encoder 12 generating an acoustically reproducible encoding of the numerical values, and reproducing the numerical values via the electro-acoustic transducer 11.

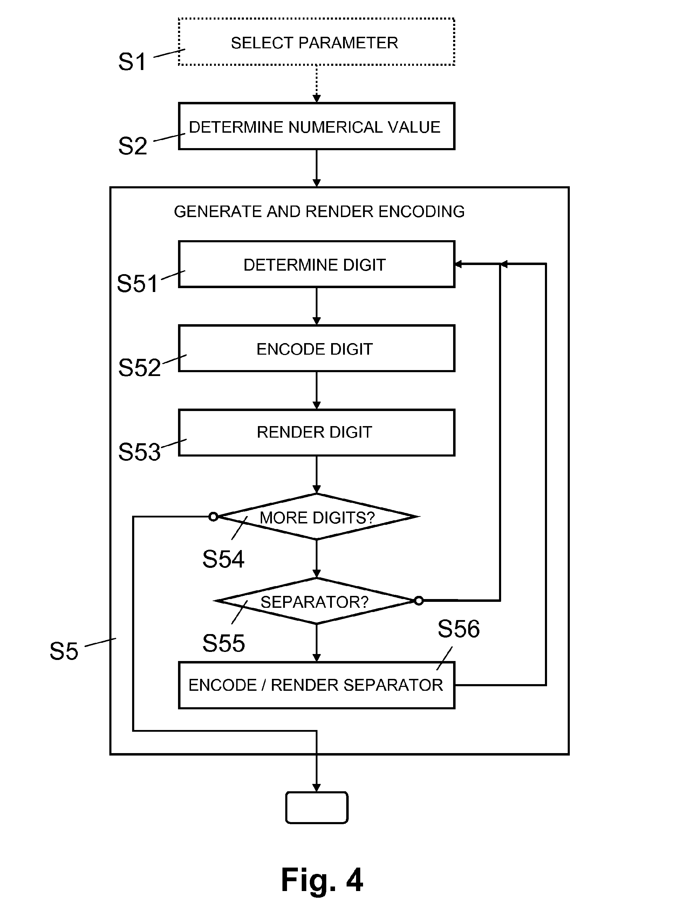

As illustrated in Figures 3 and 4, in optional step S1, a parameter 151, 152 having a numerical value is selected. For example, the parameter 151, 152 is selected by the user by way of navigating through a user interface, a menu, a parameter list, or the like, using the operating elements 10. Preferably, the navigation to and/or selection of the parameter is indicated acoustically to the user via the electro-acoustic transducer 11. For example, the control module 14 generates acoustic signals identifying unambiguously in each case a parameter 151, 152 that is defined by the current position of a cursor or pointer, and confirming selection of such a parameter 151, 152. In an embodiment, if a parameter, 151, 152, is entered, for example via scrolling by way of up/down buttons of operating elements 10, an acoustic indication, such as a "beep" of constant pitch, is generated for each numerical increment. Depending on the embodiment and/or scenario, the respective parameter 151, 152 is shown on display 15 as a label (name), a graphical symbol, and/or a numerical value.

In step S2, a numerical value is determined. For example, the numerical is determined based on the selection of a parameter performed in step S1, or based on a data entry performed by the user using the operating elements 10. The numerical value, assigned to the selected parameter or associated with the data entry, is stored in data memory of the ambulatory infusion device 1, e.g. in a parameter store or a data entry register, respectively. The numerical value is passed by the control module 14 to the audio encoder 12 by reference or data value. In an embodiment, the control module 14 enters the numerical value in an encoding buffer, where it is retrieved by the audio encoder 12, e.g. triggered by a signal from the control module 14. Depending on the embodiment and scenario, the numerical values comprise one or more digits and are entered in different formats. For example, depending on the parameter type, parts of the numerical values may be separated by a colon, e.g. "HH:MM" for time values, or a period, e.g. "NN.DD", "N.DD", or "N.D" for insulin amount values; or as numbers only, without any separators, e.g. "HHMM" or "NNDD", "NDD", "ND", "NN", or "N", etc.

In step S3, the audio encoder 12 generates an acoustically reproducible encoding of the numerical value.

In step S31, the audio encoder 12 determines a digit of the numerical value. In the case that the numerical value comprises more than one digit, processing starts preferably with the most significant digit, or alternatively, with the least significant digit.

In step S32, the audio encoder 12 generates an acoustically reproducible encoding of the digit determined in step S31. The audio encoding is performed based on the acoustic profile 13 selected and set by the user or supplier of the ambulatory infusion device 1. Tables 1 and 2 illustrate the encoding of decimal digits "1"-"9" by mapping in each case the digit to a defined sequence of successive notes of a musical scale 2 with increasing pitch, specifically a C-major scale 2, as shown in Figure 2. Tables 1 and 2 illustrate the encoding of decimal digits according to different acoustic profiles 13.

**Table 1**

| Digit | Audio encoding - standard acoustic profile |
|---|---|
| 1 | DO3 |
| 2 | DO3-RE3 |
| 3 | DO3-RE3-MI3 |
| 4 | DO3-RE3-MI3-FA3 |
| 5 | DO3-RE3-MI3-FA3-SO3 |
| 6 | DO3-RE3-MI3-FA3-SO3-LA3 |
| 7 | DO3-RE3-MI3-FA3-SO3-LA3-SI3 |
| 8 | DO3-RE3-MI3-FA3-SO3-LA3-SI3-DO4 |
| 9 | DO3-RE3-M3I-FA3-SO3-LA3-SI3-DO4-RE4 |
| 0 | DO3-LA2 |
| ./: | DO4 |

Table 1 shows the mapping of decimal digits according to an acoustic profile 13 defined as a "standard" acoustic profile, whereas Table 2 shows the mapping of decimal digits according to an acoustic profile 13 defined as a "low tone" or "low frequency" acoustic profile which is selected preferably for and/or by users with a partial hear loss handicap. Essentially, the "standard" and "low tone" acoustic profiles define the mapping or encoding of the decimal digits with different octaves, i.e. with different pitch or frequency levels. The "standard" acoustic profile shown in Table 1 defines the third octave or pitch level, whereby decimal digits "8" and "9" also use the first notes of the fourth octave"DO4" or "DO4-RE4", respectively.

The "low tone" or "low frequency" acoustic profile shown in Table 2 defines a lower frequency encoding using the second octave or pitch level, whereby decimal digits "8" and "9" also use the first two notes of the third octave "DO3" or "DO3-RE3", respectively.

**Table 2**

| Digit | Audio encoding - low tones acoustic profile |
|---|---|
| 1 | DO2 |
| 2 | DO2-RE2 |
| 3 | DO2-RE2-MI2 |
| 4 | DO2-RE2-MI2-FA2 |
| 5 | DO2-RE2-MI2-FA2-SO2 |
| 6 | DO2-RE2-MI2-FA2-SO2-LA2 |
| 7 | DO2-RE2-MI2-FA2-SO2-LA2-SI2 |
| 8 | DO2-RE2-MI2-FA2-SO2-LA2-SI2-DO3 |
| 9 | DO2-RE2-M2I-FA2-SO2-LA2-SI2-DO3-RE3 |
| 0 | DO2-LA1 |
| ./: | DO3 |

As is shown in Tables 1 and 2, the decimal digits "1" to "9" are encoded in each case by a sequence of ascending notes of the musical scale 2 (i.e. with increasing pitch), starting with the first note of the octave, i.e. the 1^{st} scale degree or tonic. The number of notes in the sequence is equal to the numerical value of the respective digit. For example, the digit "1" has a sequence of just one note, the 1^{st} note or scale degree of the octave; the digit "2" has a sequence of two notes, the 1^{st} and 2^{nd} notes or scale degrees of the octave; the digit "3" has a sequence of three notes, the 1^{st} to 3^{rd} notes or scale degrees of the octave; etc. up to the digit "9" which has a sequence of nine notes, 1^{st} to 7^{th} notes or scale degrees of the octave and the 1^{st} and 2^{nd} notes or scale degrees of the next higher octave.

As is shown in Tables 1 and 2, the digit "0" is encoded by a special sequence of notes selected from the musical scale 2. In the acoustic profiles shown in Tables 1 and 2, the digit "0" is encoded by a sequence of two successive notes with descending pitch. For example, the "standard" acoustic profile shown in Table 1 defines for the digit "0" the sequence of "DO3-LA2", whereas the "low tone" or "low frequency" acoustic profile shown in Table 2 defines for the digit "0" the sequence of "DO2-LA1 ".

In step S33, the audio encoder 12 checks whether the numerical value includes further digits to be encoded. If there are no further digits, processing continues in step S4; otherwise, processing continues in step S34.

In step S34, the audio encoder 12 checks whether the numerical value has or requires a separator to be inserted before the next digit to be encoded. If no separator is required, processing continues in step S31 by determining the next digit to be encoded; otherwise, processing continues in step S35. The audio encoding is again performed based on the selected acoustic profile 13.

In step S35, the audio encoder 12 generates an acoustically reproducible encoding of the separator that is or has to be inserted before the next digit of the numerical value. As outlined above, for time values, a colon ":" is used as a separator between the hours part and the minutes part; for amount values, a period "." is used as a separator between the integer part and the fractional part. In the examples of Tables 1 and 2, both separators ".", ":" are encoded by a single tone which has a pitch that corresponds to a note with an octave higher than the first note of the musical scale 2 used for encoding the digits "1" to "7", i.e. an octave higher than the 1^{st} scale degree or tonic. Specifically, in the "standard" acoustic profile shown in Table 1, the separators are encoded by a "DO4", whereas in the "low tone" acoustic profile shown in Table 2, the separators are encoded by a "DO3", respectively.

In the embodiment of Figure 3, the acoustically reproducible encoding of the numerical value is entered and stored digit by digit in an audio encoding output buffer 120. Table 3 illustrates an example of the content of the audio encoding output buffer 120 for the amount value of "1.05" using the "standard" acoustic profile or the "low tone" acoustic profile, respectively.

**Table3**

| | Empty digit | Most significant digit "1" | Separator "." | Digit "0" | Least significant digit "5" |
|---|---|---|---|---|---|
| Standard acoustic profile | - | "DO3" | "DO4" | "D03-LA2" | "DO3-RE3-MI3-FA3-SO3" |
| Low tone acoustic profile | - | "DO2" | "DO3 | "DO2-LA1" | "DO2-RE2-MI2-FA2-SO2" |

For example, the sequences of one or more notes are stored in the audio encoding output buffer 120 by way of corresponding digital codes.

In step S4, the audio encoder 12 renders the numerical value via the electro-acoustic transducer 11 using the acoustically reproducible encoding stored in the audio encoding output buffer 120. The audio encoder 12 is further configured to insert silent pauses in between the acoustic rendering of the digits and separator. Depending on the embodiment, the length of these pauses varies for the different acoustic profiles 13 and/or may be set by the user. Accordingly, Table 4 illustrates the sequence of tones and pauses produced for the acoustic rendering of the numerical (amount) value "1.05", using the "standard" acoustic profile (upper row) or the "low tone" acoustic profile (lower row), respectively.

**Table 4**

| "1" | | "." | | "0" | | "5" |
|---|---|---|---|---|---|---|
| "DO3 | pause | "DO4" | pause | "DO3-LA2" | pause | "DO3-RE3-MI3-FA3-SO3" |
| "DO2" | pause | "DO3 | pause | "DO2-LA1" | pause | "DO2-RE2-MI2-FA2-SO2" |

Table 5 illustrates an example of the frequency mapping for the defined tones.

**Table5**

| Tone | Frequency | Tone | Frequency | Tone | Frequency |
|---|---|---|---|---|---|
| DO2 | 528 Hz ±5 % | DO3 | 1056 Hz ±5% | DO4 | 2112 Hz ±5 % |
| RE2 | 594 Hz±5% | RE3 | 1188 Hz ±5% | RE4 | 2376 Hz ±5 % |
| MI2 | 660 Hz±5% | MI3 | 1320 Hz ±5% | MI4 | 2640 Hz ±5 % |
| FA2 | 704 Hz ±5 % | FA3 | 1408 Hz ±5 % | FA4 | 2816 Hz ±5 % |
| SO2 | 792 Hz±5% | SO3 | 1584 Hz ±5% | SO4 | 3168 Hz ±5 % |
| LA2 | 880 Hz±5% | LA3 | 1760 Hz ±5% | LA4 | 3520 Hz ±5% |
| S12 | 990 Hz±5% | SI3 | 1980 Hz ±5% | SI4 | ... |

In the embodiment of Figure 4, steps S3 and S4 of Figure 3, for generating the acoustically reproducible encoding of the numerical value and reproducing the numerical value via the electro-acoustic transducer 11, are combined in step S5 as outlined below.

In step S51, the audio encoder 12 determines a digit of the numerical value, as described above with reference to step S31 of Figure 3.

In step S52, the audio encoder 12 generates an acoustically reproducible encoding of the digit determined in step S51, as described above with reference to step S32 of Figure 3.

In step S53, the audio encoder 12 renders the digit via the electro-acoustic transducer 11 using the digit's acoustically reproducible encoding generated in step S52. The audio encoder 12 further generates a silent pause after the acoustic rendering of the digit, as described above with reference to step S4 of Figure 3.

In step S54, the audio encoder 12 checks whether the numerical value includes further digits to be encoded. If there are no further digits, processing of the numerical value ends; otherwise, processing continues in step S55.

In step S55, the audio encoder 12 checks whether the numerical value has or requires a separator to be inserted before the next digit to be encoded, as described above with reference to step S34 of Figure 3. If no separator is required, processing continues in step S51 by determining the next digit to be encoded; otherwise, processing continues in step S56.

In step S56, the audio encoder 12 generates an acoustically reproducible encoding of the separator, as described above with reference to step S35 of Figure 3. Furthermore, the audio encoder 12 renders the separator via the electro-acoustic transducer 11 using the digit's acoustically reproducible encoding generated in step S56. The audio encoder 12 further generates a silent pause after the acoustic rendering of the separator, as described above with reference to step S4 of Figure 3. Subsequently, processing continues in step S51 by determining the next digit to be encoded.

In addition to encoding and rendering acoustically numerical values such as insulin amounts, e.g. bolus-amount, basal rate-amount, etc., time of day values, e.g. infusion time, alarm clock time, etc., and/or infusion durations, e.g. for extended bolus or temporary basal rate activities, the audio encoder 12 is further used to encode and render acoustically numerical values assigned to specific objects and/or events associated with the ambulatory infusion device 1 or its operation, respectively. For example, numerical values assigned to messages, such as Error, Maintenance, Warning, or Reminder (EMWR) messages; numerical values assigned to device status values such as "running" or "stopped" pump or insulin delivery, respectively; and/or numerical values assigned to currently selected menu screens, currently selected menu functions, currently selected display objects, and/or currently activated operating elements, e.g. which button is pressed, etc.

In an embodiment, when a button is pressed while a tone sequence is being rendered, rendering of the tone sequence will be stopped. Alternatively or in addition, various messages or objects, may be configured such that they must not be interrupted by activation of operating elements, e.g. an alarm or alert message may be set non-interruptible so that rendering of the tone sequence associated with an alarm or alert message is not interrupted by the tone sequence assigned to pressing a button.

Consequently, the audio encoder 2 provides to a user of the ambulatory infusion device 1 an improved user interface making it possible for a user who has visual impairment and/or partial hearing loss to easily and intuitively program, operate, and obtain feedback from the ambulatory infusion device 1. Whatever action is taken in operating the ambulatory infusion device 1, the ambulatory infusion device 1 provides to its user corresponding audio output to ensure safe menu navigation. The user does not have to rely on "external" help to operate the ambulatory infusion device 1 and memorizing the principle of menu navigation becomes much easier and more intuitive. The user is always informed about the current status/reaction/problem of the ambulatory infusion device 1 as well as numerical values entered and/or stored in the ambulatory infusion device 1.

As a further option, the ambulatory infusion device 1 is further configured to provide vibration feedback, in addition to acoustic signalization, e.g. depending on a user setting and/or selected active acoustic profile 13.

It should be noted that, in the description, the computer program code has been associated with specific functional modules and the sequence of the steps has been presented in a specific order, one skilled in the art will understand, however, that the computer program code may be structured differently and that the order of at least some of the steps could be altered, without deviating from the scope of the invention.

## Claims

1. An ambulatory infusion device (1), comprising:
an electro-acoustic transducer (11); and
an audio encoder (12) connected to the electro-acoustic transducer (11) and configured to generate an acoustically reproducible encoding of numerical values, and to signal the numerical values via the electro-acoustic transducer (11);
wherein the audio encoder (12) is configured to receive a numerical value and to generate subsequently the acoustically reproducible encoding by representing a digit of the numerical value with a sequence of successive notes of a musical scale (2), the sequence comprising a quantity of notes corresponding to a value of the digit.

2. The device (1) of claim 1, wherein the device (1) further comprises different user selectable acoustic profiles (13), each acoustic profile (13) defining a pitch level of the scale (2); and the audio encoder (12) is configured to signal the numerical values using the pitch level defined in the acoustic profile (13) selected by the user.

3. The device (1) of claim 2, wherein the different acoustic profiles (13) further define different durations of the notes; and the audio encoder (12) is configured to signal the numerical values using the durations of the notes defined in the acoustic profile (13) selected by the user.

4. The device (1) of one of claims 1 to 3, wherein the audio encoder (12) is further configured to generate an acoustically reproducible encoding of numerical values having more than one digit, by generating a pause of defined duration between representations of two successive digits.

5. The device (1) of one of claims 1 to 4, wherein the audio encoder (12) is further configured to generate an acoustically reproducible encoding of a numerical infusion amount value having an integer part and a fractional part, by generating a defined tone between representations of the integer part and the fractional part.

6. The device (1) of one of claims 1 to 5, wherein the audio encoder (12) is further configured to generate an acoustically reproducible encoding of numerical time of day values having a two-digit hours part and a two-digits minutes part, by generating a defined tone between representations of the hours part and the minutes part.

7. The device (1) of one of claims 1 to 5, wherein the device (1) further comprises a control module (14) configured to receive commands from a user, and to select based on the user commands parameters (151, 152) stored in the device (1); and the audio encoder (12) is further configured to generate an acoustically reproducible encoding of numerical values of the parameters (151, 152) selected based on the user commands.

8. A method of signaling acoustically numerical values to a user of an ambulatory infusion device (1), the method comprising:
receiving in the ambulatory infusion device (1) the numerical values;
generating (S3) subsequently in the ambulatory infusion device (1) an acoustically reproducible encoding of the numerical values received; and
signaling (S4) the numerical values via an electro-acoustic transducer (11);
wherein the acoustically reproducible encoding is generated (S3) by representing a digit of a numerical value with a sequence of successive notes of a musical scale (2), the sequence comprising a quantity of notes corresponding to a value of the digit.

9. The method of claim 8, wherein the method further comprises storing in the ambulatory infusion device (1) different user selectable acoustic profiles (13), each acoustic profile (13) defining the pitch level of the scale (2); and the numerical values are signaled (S4) using the pitch level defined in the acoustic profile (13) selected by the user.

10. The method of claim 9, wherein the different acoustic profiles (13) are stored with a definition of different durations of the notes; and the numerical values are signaled (S4) using the durations of the notes defined in the acoustic profile (13) selected by the user.

11. The method of one of claims 8 to 10, wherein generating (S3) an acoustically reproducible encoding of numerical values having more than one digit includes generating a pause of defined duration between representations of two successive digits.

12. The method of one of claims 8 to 11, wherein generating (S3) an acoustically reproducible encoding of a numerical infusion amount value, having an integer part and a fractional part, includes generating a defined tone between representations of the integer part and the fractional part.

13. The method of one of claims 8 to 12, wherein generating (S3) an acoustically reproducible encoding of numerical time of day values, having a two-digit hours part and a two-digits minutes part, includes generating a defined tone between representations of the hours part and the minutes part.

14. The method of one of claims 8 to 13, wherein the method further comprises receiving user commands; selecting based on the user commands parameters stored in the device (1); and generating (S3) an acoustically reproducible encoding of the numerical values of the parameters selected based on the user commands.

15. A computer program product comprising a tangible computer readable medium having stored thereon computer program code configured to direct a processor of an ambulatory infusion device (1), which includes an electro-acoustic transducer (11), such that the processor
receives in the ambulatory infusion device (1) a numerical value;
generates (S3) subsequently in the ambulatory infusion device (1) an acoustically reproducible encoding of the numerical value, by representing a digit of the numerical value with a sequence of successive notes of a musical scale (2), the sequence comprising a quantity of notes corresponding to a value of the digit; and
signals (S4) the numerical values via the electro-acoustic transducer (11).
